# EUROPÄISCHE PATENTSCHRIFT

(11) **EP 1 588 728 B1**
(45) Veröffentlichungstag und Bekanntmachung des Hinweises auf die Patenterteilung: **01.06.2016**
(21) Anmeldenummer: 04023737.2
(22) Anmeldetag: 06.10.2004
(51) Int. Cl.: A61M 5/145, A61M 5/44

(54) **Vorrichtung zur Injektion einer Flüssigkeit aus einer Spritze mit einer beheizbaren Halterung für die Spritze**
Device for injecting a liquid from a syringe with a heated holder for the syringe
Dispositif d'injection d'un liquide d'une seringue avec porteur chauffé pour la seringue

(30) Priorität: 21.04.2004 DE 202004006479 U
(43) Veröffentlichungstag der Anmeldung: 26.10.2005
(73) Patentinhaber: Medtron AG, 66128 Saarbrücken (DE)
(72) Erfinder: Koch, Wolfgang, 66584 Spiesen (DE); Altmeyer, Hanno, 66538 Neunkirchen (DE)
(74) Vertreter: Wagner, Matthias

(56) Entgegenhaltungen:
- WO-A-01/08727
- GB-A- 1 446 412
- US-A- 5 520 653
- US-A1- 2002 022 807
- US-A1- 2003 120 212
- US-A1- 2004 024 361

## Beschreibung

Die Erfindung bezieht sich auf eine Vorrichtung zur Injektion einer Flüssigkeit aus einer Spritze mit einem Kolben, einer Betätigungseinrichtung, welche mit dem aus der Spritze herausragenden Ende des Kolbens in einer Kolbenaufnahme lösbar verbindbar ist, sowie einer Halterung für die Spritze, wobei die Kolbenaufnahme der Betätigungseinrichtung und die Halterung für die Spritze an einem Tragteil befestigt sind und die Halterung der Spritze beheizbar ausgebildet ist.

Vorrichtungen zur Injektion von Flüssigkeiten sind beispielsweise aus der EP 0 587 567 B1, EP 0 584 531 B1, EP 0 171 009 B1 oder EP 0 346 950 B2 bekannt. Hierbei wird im allgemeinen die Betätigungsvorrichtung mit der Spritze durch axiales Einstecken und nachfolgendes Verdrehen der Spritze befestigt, während das Lösen der Spritze in umgekehrter Reihenfolge stattfindet. Hierbei ist es auch bekannt, die Spritze in ein rohrförmiges Gehäuse aus kompaktem Kunststoff einzusetzen, welches ebenfalls am Tragteil befestigt wird. Diese rohrförmige Ummantelung dient der Stabilisierung und Druckhaltung während des Injektionsvorganges.

Vorrichtungen der erfindungsgemäßen Art werden beispielsweise im Bereich der Computertomographie eingesetzt, um Kontrastmittel zu spritzen. Solche Kontrastmittel müssen eine hohe Viskosität aufweisen und sind darüber hinaus auf nahezu Körpertemperatur vorzuwärmen. Wenn man mit Spritzen größeren Inhaltes arbeitet, aus denen nacheinander kleinere Mengen verabreicht werden, verbleiben Restmengen des Kontrastmittels in der Spritze und diese müssen dann über einen längeren Zeitraum auf nahezu Körpertemperatur - ca. 37°C - gehalten werden. Üblich ist es, die Spritzen mit zusätzlichen äußeren Heizmanschetten zu versehen, die man um die Spritze klammert und welche dann elektrisch über Kabel an entsprechende Energiequellen angeschlossen werden. Bei Entfernen der Spritze sind diese Heizmanschetten dann wiederum erst zu entfernen.

Aus der US 2003/0120212 A1 und der US 2004/0024361 A1 sind gattungsgemäße Vorrichtung bekannt, bei denen die Beheizung der Spritzen über eine an der Halterung der Spritze befestigte äußere Heizmanschette erfolgt. Zwar braucht hierbei die Heizmanschette aufgrund ihrer Befestigung an der Halterung nicht mehr aufwendig an der Spritze befestigt bzw. von dieser gelöst zu werden, jedoch ist die Heizmanschette offen zugänglich, damit anfällig für Beschädigungen und behindert das schnelle Einlegen einer Spritze in die Halterung.

Die GB 1,446,412 beschreibt eine Temperiereinrichtung für Infusionsschläuche.

Der Erfindung liegt die Aufgabe zugrunde, eine Vorrichtung zu schaffen, mit der die Spritzen, welche sich in der Injektionsvorrichtung befinden, über einen längeren Zeitraum auf der gewünschten Verarbeitungstemperatur gehalten werden können, wobei die eingesetzte Heizung geschützt vor Beschädigung und äußerem Zugriff sein soll und die Handhabung der Vorrichtung nicht beeinträchtigt.

Erfindungsgemäß wird diese Aufgabe bei einer gattungsgemäßen Vorrichtung zur Injektion einer Flüssigkeit aus einer Spritze dadurch erreicht, daß die Halterung aus einem wärmeleitfähigen Material gefertigt ist und als oberseitig offene Schale mit zylindrischer Auflagefläche für die Spritze ausgebildet ist, in welche die Spritze durch Verändern ihrer Position quer zu ihrer Längsachse und zur Längsachse der Schale einlegbar ist und auf der der Kolbenaufnahme der Betätigungseinrichtung zugewandten Seite mit einem Flansch zum Befestigen an dem Tragteil ausgebildet ist und in dem Flansch Taschen ausgebildet sind, in die elektrische Heizwiderstände eingebaut sind.

Erfindungsgemäß wird also die Halterung der Spritze direkt beheizt. Es entfällt daher das zusätzliche Element einer Heizmanschette, welche um die Spritze gelegt werden muß und nach Beendigung der Injektionsvorgänge wieder von der Spritze entfernt werden muß. Vorteilhafte Weiterbildungen der erfindungsgemäßen beheizbaren Halterung sind den kennzeichnenden Merkmalen der Unteransprüche entnehmbar.

Durch die erfindungsgemäße beheizbare Halterung der Spritze wird das Handling beim Wechseln der Spritze wesentlich vereinfacht, es sind keine zusätzlichen Teile sowie äußere Verkabelungen zur Stromversorgung notwendig. Der elektrische Anschluß kann über die Spritzenhalterung und das Tragteil erfolgen.

Erfindungsgemäß wird vorgeschlagen, die Halterung aus einem wärmeleitfähigen Material, wie beispielsweise Aluminium, zu fertigen. Die Halterung ist mit einer elektrischen Heizeinrichtung versehen, die in die Halterung eingebaut ist. Als Heizeinrichtung werden elektrische Widerstandsheizungen verwendet, die vorteilhaft mit Niederspannung betrieben werden. Als Widerstandsheizung kommen insbesondere Heizdrähte, Heizbänder, Heizfasern aus elektrisch leitfähigen Materialien in Frage, wobei neben Kupfer auch beispielsweise Kohlefasern eingesetzt werden können. Bevorzugt ist die Widerstandsheizung als elektrischer Widerstand ausgebildet und mindestens ein solcher elektrischer Widerstand in die Halterung der Spritze eingebaut. Die Heizeinrichtung ist bevorzugt regelbar ausgebildet, wozu die Halterung mit mindestens einem Temperaturmeßfühler zum Erfassen der Temperatur der Halterung ausgestattet ist.

Hierbei wird vorgeschlagen, in die Halterung nahe der Anordnung der elektrischen Heizeinrichtung, z. B. der Widerstände, mindestens einen Sensor zum Erfassen der Temperatur der Halterung einzusetzen. Zum Erreichen und Einhalten der gewünschten Temperatur ist eine Regeleinrichtung vorgesehen, welcher die von den Sensoren ermittelte Temperatur zugeführt wird, wobei die den elektrischen Widerständen zuführbare Spannung zwecks Beheizung der Halterung der Spritze in Abhängigkeit von der gemessenen Temperatur der Halterung so gesteuert wird, daß die Temperatur der sich in der Spritze befindenden zu injizierenden Flüssigkeit einen vorgegebenen Wert nicht übersteigt. Die Länge der Halterung der Spritze sollte etwa die halbe Länge der Spritze überdecken. Die Wärme wird dann von der Spritzenhalterung auf die Spritze und die sich in der Spritze befindende Flüssigkeit übertragen.

Die Form der Halterung der Spritze kann unterschiedlich je nach Art der Befestigung derselben einerseits am Tragteil für die Betätigungseinrichtung - den sogenannten Injektor - und/oder der Art der Befestigung der Spritze in der Halterung ausgebildet sein. Gemäß der Erfindung ist vorgesehen, die Halterung der Spritze so auszugestalten, daß die Spritze quer zur Längsachse der Spritze in die Halterung einlegbar ist und entfernbar ist. Hierbei ist die Halterung für die Spritze als ein einseitiges, insbesondere oberseitig offenes Lager ausgebildet, in welches die Spritze durch Verändern ihrer Position quer zu ihrer Längsachse und zur Längsachse des Lagers einlegbar und wieder entfernbar ist. Das Lager ist als Schale mit zylindrischer Auflagefläche für die Spritze ausgebildet. Das Lager sollte mindestens den halben Durchmesser der Spritze umschließen. Bevorzugt ist die das Lager für die Spritze bildende Halbschale seitlich durch sich tangential anschließende gerade verlaufende Schalenwände vergrößert, so daß die Spritze in dem Lager versenkbar ist.

Die Halterung der Spritze ist an der der Kolbenaufnahme der Betätigungseinrichtung zugewandten Seite mit einem Flansch zum Befestigen am Tragteil ausgebildet.

Auch der Einbau von Heizpatronen ist möglich. Damit ist auch ein einfacher elektrischer Anschluß der Heizeinrichtung, d.H. der Heizwiderstände, über das Tragteil ermöglicht. Bei Entfernen der Spritze aus der Halterung verbleibt die Heizeinrichtung einschließlich ihrer elektrischen Anschlüsse in Funktion und muß nicht entfernt werden.

Ein Ausführungsbeispiel der Erfindung wird nachfolgend anhand der Zeichnung erläutert. Es zeigen:

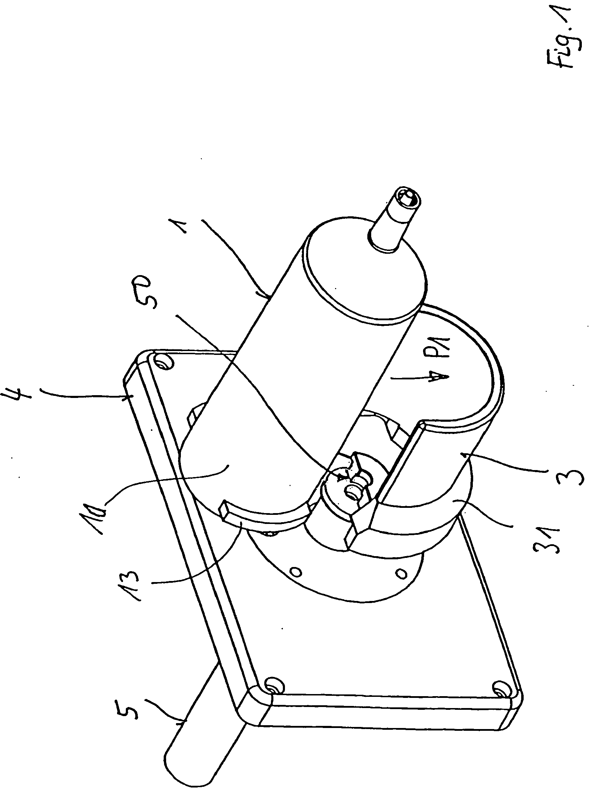

- Figur 1: eine perspektivische Darstellung einer Vorrichtung zur Injektion einer Flüssigkeit mit einer Spritze,
- Figur 2: die beheizbare Halterung der Spritze in perspektivischer Ansicht

Die Vorrichtung zum Injizieren einer Flüssigkeit mittels einer Spritze 1 umfaßt ein Tragteil 4, beispielsweise eine Tragplatte mit der Betätigungseinrichtung 5, welche eine Kolbenaufnahme 50 aufweist, die an dem Tragteil 4 befestigt ist. Des weiteren ist an dem Tragteil 4 auf der die Kolbenaufnahme 50 aufweisenden Seite dieser zugeordnet die Halterung 3 für die Spritze 1 in Gestalt einer nach oben offenen Lagerschale 3 angebracht. Die Lagerschale 3 weist hierbei an der dem Tragteil 4 zugewandten Seite einen Flansch 31 auf, mit dem sie an dem Tragteil 4 beispielsweise mittels Schrauben oder Bolzen befestigbar ist.

Die Spritze 1 ist mit ihrem hinteren Endbereich 10 in die offene Lagerschale 3 von oben her in Pfeilrichtung P1 quer zu ihrer Längserstreckung einsetzbar, wobei sie mit ihrer Wandung an der Innenoberfläche der Lagerschale 3 zur Anlage kommt. Die Spritze 1 weist an ihrem hinteren Ende außenseitig Vorsprünge 13 auf, mit welchen sie in entsprechenden Nuten der Lagerschale 3 im Bereich des Flansches 31 geführt ist.

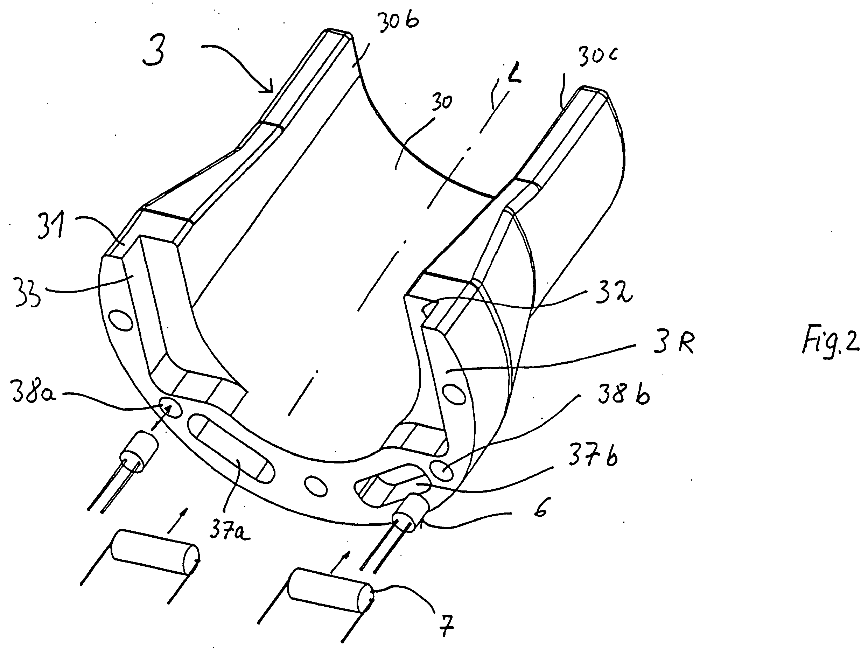

In der Figur 2 ist die beispielsweise als offenes Lager in Gestalt einer Lagerschale ausgebildete Halterung 3 in weiteren Einzelheiten dargestellt. Die Lagerschale 3 hat die Form einer Halbschale 30 mit einer zylindrischen Auflagerfläche und sie ist an ihren beiden Längsseiten durch seitliche Wände 30b, 30c, die tangential gerade verlaufend sich nach oben erstrecken, vergrößert, so daß eine eingelegte Spritze mindestens auf der Hälfte ihrer Gesamtlänge und mindestens der Hälfte ihres Umfanges von der Halterung seitlich umfaßt wird. An dem rückwärtigen Ende 3R, d. h. an der dem Tragteil 4 zugewandten Befestigungsseite der Halterung ist die Lagerschale 3 durch einen Flansch 31 vergrößert. In dem Flansch sind von der offenen Oberseite des Lagers her symmetrisch zueinander Nuten 32, 33 ausgebildet, in denen die eingelegte Spritze 1 mit ihren Vorsprüngen 13 geführt ist und in dem Lager 3 um die Längsachse L drehbar ist.

Von der Rückseite 3R her sind in der Halterung im Bereich des Flansches 31 zwei Taschen 37a und 37b ausgebildet, in welche je ein Heizwiderstand 7 eingesetzt wird. Die Heizwiderstände 7 werden bevorzugt in die Taschen eingegossen. Seitlich neben den in die Halterung 3 eingesetzten Heizwiderständen 7 sind Sacklöcher 38a und 38b vorgesehen, in welche je ein Temperatursensor 6 eingesetzt wird, welche die Temperatur der Halterung 3 erfassen.

Die Heizwiderstände 7 werden von einer nicht dargestellten Regeleinrichtung mittels einer geregelten Spannung erhitzt, uns zwar soweit, daß die gesamte Halterung 3 der Spritze auf eine definierte Temperatur von etwa 37°C innerhalb weniger Minuten gebracht wird. Da die Spritze 1 mit ihrer Wandung an der solchermaßen beheizten Halbschale 30 anliegt, findet ein rascher Wärmeübergang zur Temperierung der in der Spritze 1 befindlichen Flüssigkeit statt. Die Temperatursensoren 6 messen hierbei die erreichte Temperatur und geben die Werte an die Regeleinrichtung weiter. Bei Erreichen einer maximalen Temperatur von 42°C wird die an den Heizwiderständen 7 anliegenden Spannung und damit die Beheizung abgeschaltet. Nach Abkühlen der Halterung 3 auf einen unteren Wert kann dann die Beheizung wieder eingeschaltet werden, entweder automatisch oder von Hand. Durch die gewählte Form der Halterung der Spritze ist auch ein gleichmäßiger Wärmeübergang im Bereich der Halterung 3 ermöglicht.

## Patentansprüche

1. Vorrichtung zur Injektion einer Flüssigkeit aus einer Spritze (1) mit einem Kolben, die Vorrichtung umfassend eine Betätigungseinrichtung (5), welche mit dem aus der Spritze (1) herausragenden Ende des Kolbens in einer Kolbenaufnahme (50) lösbar verbindbar ist, sowie eine Halterung (3) für die Spritze, wobei die Kolbenaufnahme (50) der Betätigungseinrichtung (5) und die Halterung (3) für die Spritze an einem Tragteil (4) befestigt sind und die Halterung (3) der Spritze (1) beheizbar ausgebildet ist, **dadurch gekennzeichnet, dass** die Halterung (3) aus einem wärmeleitfähigen Material gefertigt ist und als oberseitig offene Schale (30) mit zylindrischer Auflagefläche für die Spritze (1) ausgebildet ist, in welche die Spritze (1) durch Verändern ihrer Position quer zu ihrer Längsachse und zur Längsachse (L) der Schale (30) einlegbar ist und auf der der Kolbenaufnahme (50) der Betätigungseinrichtung (5) zugewandten Seite mit einem Flansch (31) zum Befestigen an dem Tragteil (4) ausgebildet ist und in dem Flansch (31) Taschen (37a, 37b) ausgebildet sind, in die elektrische Heizwiderstände (7) eingebaut sind.

2. Vorrichtung nach Anspruch 1, **dadurch gekennzeichnet, dass** in den Flansch (31) nahe der Position der elektrischen Heizwiderstände (7) mindestens ein Sensor (6) zum Erfassen der Temperatur der Halterung (3) eingesetzt ist.

3. Vorrichtung nach einem der Ansprüche 1 oder 2, **dadurch gekennzeichnet, dass** eine Regeleinrichtung vorgesehen ist, welcher die von den Sensoren (6) ermittelte Temperatur zuführbar ist, wobei die den elektrischen Heizwiderständen (7) zuführbare Spannung zwecks Beheizung in Abhängigkeit von der gemessenen Temperatur der Halterung (3) steuerbar ist, so dass die Temperatur der sich in der Spritze (1) befindenden zu injizierenden Flüssigkeit einen vorgegebenen Wert nicht übersteigt.

4. Vorrichtung nach einem der Ansprüche 1 bis 3, **dadurch gekennzeichnet, dass** die Heizwiderstände (7) mit Niederspannung betrieben werden.

5. Vorrichtung nach einem der Ansprüche 1 bis 4, **dadurch gekennzeichnet, dass** die Schale (30) mindestens den halben Umfang der Spritze (1) umschließt.

6. Vorrichtung nach einem der Ansprüche 1 bis 5, **dadurch gekennzeichnet, dass** die Schale (30) für die Spritze (1) seitlich durch sich tangential anschließende gerade verlaufende Schalenwände (30b, 30c) vergrößert ist.

7. Vorrichtung nach einem der Ansprüche 1 bis 6, **dadurch gekennzeichnet, dass** die Schalenwände (30b, 30c) entlang ihrer oberen Ränder zur Vergrößerung der Einführöffnung abgeschrägt sind.

8. Vorrichtung nach einem der Ansprüche 1 bis 7, **dadurch gekennzeichnet, dass** die Schale (30) für die Spritze (1) eine zur Aufnahme von mindestens der halben Länge der Spritze (1) ausreichende Länge aufweist.

## Claims

1. A device for injecting a liquid from a syringe (1) with a plunger, the device comprising an actuating device (5) that can be detachably connected in a plunger accommodating portion (50) to the end of the plunger protruding from the syringe (1), and a holder (3) for the syringe, wherein the plunger accommodating portion (50) of the actuating device (5) and the holder (3) for the syringe are attached to a supporting part (4) and the holder (3) of the syringe (1) is configured to be heatable, **characterised in that** the holder (3) is made from a heat-conductive material, and is configured as a tray (30) open at the top with a cylindrical support surface for the syringe (1), into which the syringe (1) can be inserted by changing its position transverse to its longitudinal axis and the longitudinal axis (L) of the tray (30), and, on the side facing towards the plunger accommodating portion (50) of the actuating device (5), is configured with a flange (31) for attachment to the supporting part (4), and pockets (37a, 37b), into which electrical heating resistors (7) are fitted, are formed in the flange (31).

2. The device according to claim 1, **characterised in that** at least one sensor (6) for detecting the temperature of the holder (3) is inserted into the flange (31) close to the position of the electrical heating resistors (7).

3. The device according to any one of the claims 1 or 2, **characterised in that** a regulating device is provided that can be supplied with the temperature determined by the sensors (6), wherein the voltage that can be supplied to the electrical heating resistors (7) can be controlled depending on the measured temperature of the holder (3) for the purpose of heating, so that the temperature of the liquid to be injected, which is located in the syringe (1), does not exceed a predefined value.

4. The device according to any one of the claims 1 to 3, **characterised in that** the heating resistors (7) are operated with low voltage.

5. The device according to any one of the claims 1 to 4, **characterised in that** the tray (30) encloses at least half the circumference of the syringe (1).

6. The device according to any one of the claims 1 to 5, **characterised in that** the tray (30) for the syringe (1) is laterally enlarged by tangentially adjacent tray walls (30b, 30c) extending in a straight line.

7. The device according to any one of the claims 1 to 6, **characterised in that** the tray walls (30b, 30c) are bevelled along their upper edges in order to enlarge the insertion opening.

8. The device according to any one of the claims 1 to 7, **characterised in that** the tray (30) for the syringe (1) has a length sufficient for accommodating at least half the length of the syringe (1).

## Revendications

1. Dispositif pour l'injection d'un liquide à partir d'une seringue (1) avec un piston, lequel dispositif comprend un dispositif d'actionnement (5), qui peut être assemblé de manière amovible à l'extrémité du piston qui dépasse hors de la seringue (1) dans un logement de piston (50), ainsi qu'un support (3) pour la seringue, le logement de piston (50) du dispositif d'actionnement (5) et le support (3) pour la seringue étant fixés à une partie porteuse (4) et le support (3) de la seringue (1) étant conçu de manière à pouvoir être chauffé, **caractérisé en ce que** le support (3) est fabriqué en un matériau conducteur de chaleur et est conçu comme une coque (30) ouverte sur le dessus avec une surface d'appui cylindrique pour la seringue (1), coque dans laquelle la seringue (1) peut être placée en modifiant sa position transversalement à son axe longitudinal et à l'axe longitudinal (L) de la coque (30), **en ce qu'**il est conçu avec une collerette (31) sur le côté proche du logement de piston (50) du dispositif d'actionnement (5) pour la fixation à la partie porteuse (4) et **en ce que** des poches (37a, 37b) dans lesquelles sont insérées des résistances chauffantes électriques (7) sont conçues dans la collerette (31).

2. Dispositif selon la revendication 1, **caractérisé en ce qu'**au moins un capteur (6) pour la détection de la température du support (3) est placé dans la collerette (31) près de l'emplacement des résistances chauffantes électriques (7).

3. Dispositif selon l'une des revendications 1 ou 2, **caractérisé en ce qu'**il est prévu un dispositif de régulation auquel peut être envoyée la température déterminée par les capteurs (6), la tension qui peut être envoyée aux résistances chauffantes électriques (7) en vue du chauffage pouvant être commandée en fonction de la température mesurée du support (3) de telle sorte que la température du liquide à injecter qui se trouve dans la seringue (1) ne dépasse pas une valeur prescrite.

4. Dispositif selon l'une des revendications 1 à 3, **caractérisé en ce que** les résistances chauffantes (7) fonctionnent en basse tension.

5. Dispositif selon l'une des revendications 1 à 4, **caractérisé en ce que** la coque (30) entoure au moins la moitié de la circonférence de la seringue (1).

6. Dispositif selon l'une des revendications 1 à 5, **caractérisé en ce que** la coque (30) pour la seringue (1) est agrandie latéralement par des parois de coque (30b, 30c) qui s'y rattachent tangentiellement et qui s'étendent de manière rectiligne.

7. Dispositif selon l'une des revendications 1 à 6, **caractérisé en ce que** les parois de coque (30b, 30c) sont biseautées le long de leurs bords supérieurs pour agrandir l'ouverture d'introduction.

8. Dispositif selon l'une des revendications 1 à 7, **caractérisé en ce que** la coque (30) pour la seringue (1) a une longueur suffisante pour recevoir au moins la moitié de la longueur de la seringue (1).
